# EUROPEAN PATENT APPLICATION

(11) **EP 2 044 944 A1**
(43) Date of publication of application: **08.04.2009**
(21) Application number: 07794053.4
(22) Date of filing: 15.06.2007
(51) Int. Cl.: A61K 31/662, A61K 31/195, A61K 33/00, A61K 9/08, A61P 25/28

(54) **NOOTROPIC MEDICINAL AGENT**

(30) Priority: 05.07.2006 RU 2006124117
(71) Applicant: Rasnetsov, Lev Davidovich, Nizhny Novgorod, 603000 (RU)
(72) Inventor: SHVARTSMAN, Iakov Yudelevich, Nizhny Novgorod, 603105 (RU); YASHNOVA, Olga Konstantinovna, Nizhny Novgorod, 603016 (RU); MELNIKOVA, Nina Borisovna, Nizhny Novgorod, 603074 (RU); PETRYAKOVA, Olga Vladimirovna, Nizhny Novgorod, 603044 (RU); GULYAEV, Ivan Valeryevich, Nizhny Novgorod, 603146 (RU)
(74) Representative: HOFFMANN EITLE
(86) International application number: PCT/RU2007/000326
(87) International publication number: WO 2008/004908

(57) **Abstract**

The invention relates to medicine, in particular to neurology and psychiatry and can be used in the form of an agent for normalising the physiological and functional activity of the central nervous system of a brain intellectually-mnestic function. The inventive medicinal agent exhibits a nootropic activity an comprises dimephosphone in the form of an active substance. Said medicinal agent is embodied in the form of an aqueous solution and also comprises citric acid and lithium carbonate at the following component ratio: 5.0-30.0 mass% dimephosphone, 0.5-5.0 mass % lithium carbonate, 3.0-4.0 mass % citric acid, the rest up to 100% being deionised water. The medicinal agent in the form of a syrup has the following component ratio: 3.0-5.0 mass% dimephosphone, 1.0-1.2 mass% lithium carbonate, 4.0-5.0 mass% citric acid and 89-92.0 mass% sixty-four percentage sugar syrup. A syrup having a high concentration of dimephosphone and a glycerin-containing syrup are also disclosed as the variants of the invention. Said medicinal agents have more physiological pH values exhibited within an extended range of concentrations, are **characterized by** the high dilution stability of the solutions thereof and have a delectable flavor, thereby easing the use thereof in a child treatment form.

## Description

### Technical Field

The invention relates to medicine, particularly to neurology and psychiatry and can be used as an agent for normalizing the physiological and functional activity of the central nervous system (CNS), brain intellectually - mnestic functions.

### Prior Art

Currently known are nootropic agents for treating various forms of intellectual insufficiency. In spite of a great arsenal of nootropic agents, main preparations in the treatment of patients with intellectual insufficiency are pyracetam (nootropyl), pantogam, glycine, (Cf. The Register of Medicinals of Russia). The known agents produce therapeutica effects only in cases of long-term use, more exactly, have a remarkably long latent period of action; therefore, elaboration of new effective and quick-acting medicinals normalizing the physiological and functional activity of CNS, the intellectually-mnestic functions of the brain is of current interest.

Also, known is the use of dimephosphone as a vasoactive agent normalizing the functions of the central nervous system (Cf. USSR Inventorship Certificate No 1754111, M., Cl. A61K31/66, published 1992). Inclusion of a preparation in the complex of medicinal therapy in the form of a 15% aqueous solution, ingestible, thrice a day, in a daily dosage of 30 mg/kg of a body weight ameliorates a course of diseases in the patients with acute and chronic disorders of cerebral and spinal circulation, craniocerebral traumas, neurooncology, hydrocephaly, migraine syndromes and reduces the time that they stay in hospital and invalidation.

Further known is a method of preventive treatment of relapses in alcoholism cases (cf. patent RF No 2236230, M., Cl. A61K31/662, published 2004) comprising monotherapy with dimephosphone, a daily dosage of 15 to 60 mg/kg of a body weight, intravenously during 15-20 days, a course of duration up to 10-15 months. The use of a preparation relieves abstinence syndrome in alcoholism cases already in the earlier stages of development, contributes to reducing a neurological deficit, normalizing psychic status thereby to raise the efficacy of treatment.

The closest to the invention per se is a method of correction of intellectually-mnestic functions in children with psychic retardation (cf. patent RF No 2020938, M., Cl. A61K31/21, published 1994), including dimephosphone therapy. A preparation is administered in the form of a 15% aqueous solution, thrice a day, in a daily dosage of 50-90 mg/kg of a body weight. The use of the preparation allows for performing correction of the intellectually-mnestic functions of the brain in diseases and conditions accompanied by memory deterioration and the decline of intellectual productivity, specifically with certain kinds of psychic disontogenesis in children.

A medicinal agent usable in analogs and a prototype is a 15% dimephosphone solution (dimethyl ester of 1,1-dimethyl-3-oxobutylphosphonic acid) and performs the functions of a nootropic preparation in addition to the known antiacidotic and membrano-stabilizing action.

However, substance - dimephosphone in aqueous media is partially hydrolysable. As a result of hydrolysis a pH value is varied up to 2.6-2.7 for a time. Alongside the bitter taste of a preparation, its strong acidity complicates its use in child's practice.

Besides, an examination of a hydrolysis at various concentrations of dimephosphone in water goes to show that only in certain ranges of concentrations, the dimephosphone exhibits buffer properties leading to a strictly well-defined pH value.

### Brief Description of Drawings

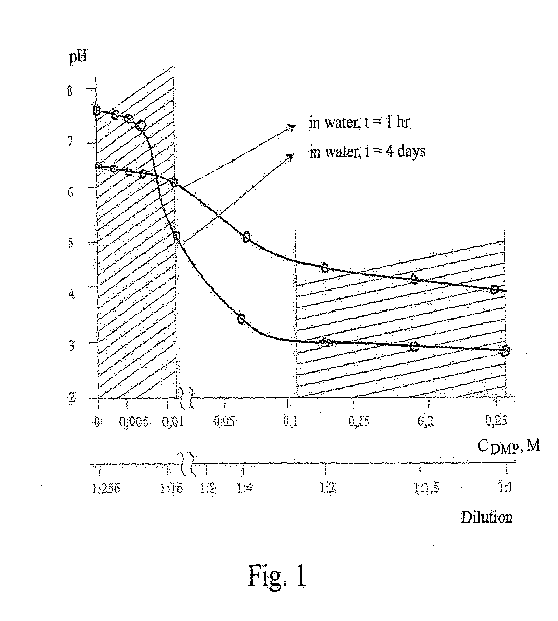

The invention will now be described with reference to the drawings and tables, wherein: Fig. 1 - curve of the dependence of a dimephosphone pH value on concentrations. The crosshatched zone-buffering area;
Table 1 - formulations of the medicinal agents proposed;
Table 2 - evaluation results of the cognitive-mnestic peculiarities of animals in an experiment;
Table 3 - evaluation results of the behavioral activity of animals while giving a test in a plant "open field";
Table 4 - results of an influence exerted by test medicinal agents on the dynamics of forming a conditioned reflex of active avoidance (CRAA) in rats;
Table 5 - results of checking produced CRAA retention in rats made 5 days after the last training session.

The curve of the dependence of a dimephosphone pH value on concentrations, as shown in Fig. 1, is indicative of instability of the pH of dimephosphone solutions to dilution in the range of concentrations of from 10 to 20% (dilution 1:4 - 1:16), which fact likewise makes difficult its use in the treatment of nervous diseases.

### Disclosure of the Invention

The invention is directed to the task whose solution consists in creating the group of effective medicinal agents in the form of aqueous solutions and syrups for normalizing the physiological and functional activity of the central nervous system (CNS), intellectually-mnestic functions of the brain, based on an active substance - dimephosphone having more physiologically active pH values and contributing to a more effective stabilization of a membrane.

Said result is achieved owing to the fact that a medicinal agent having a nootropic activity, comprising an active substance - dimephosphone and water, according to a first alternative embodiment of the invention, further contains citric aid and lithium carbonate, with the following ratio of components, wt%:

| | |
|---|---|
| dimephosphone | 15.0-30.0 |
| lithium carbonate | 0.5-5.0 |
| citric acid | 3.0-4.0 |
| water, deionized | up to 100 |

Introduction of lithium carbonate and citric acid into a dimephosphone solution results in forming new products - lithium citrate and a lithium derivative of dimephosphone.

The lithium citrate so obtained and lithium dimephosphone derivative contribute to forming buffer solutions with more physiologically effective pH values (5-7) and a more effective stabilization of a membrane.

It is known that lithium ions have psychotropic action and perform particularly well in child's neurology /Mel'nik V.A.. Mel'nik A.I. Achievements in use of lithium salts in clinical pediatrics and further perspectives in this field. //Pediatrics, 1998, No 12, pp. 76-79). Besides, the lithium ions are normotimic means to demonstrate positive psychotropic properties; do not oppress a memory condition and mental activity. Investigations of an influence exerted by dimephosphone, lithium ions in the presence of citric acid (lithium citrate) on a membrane condition and also an examination of a buffer effect of said dimephosphone in the presence of lithium ions go to show that the medicinal agent thus obtained shows a synergistic effect, more exactly, high lipophility, and the dimephosphone and its lithium derivatives easily pass histohematic barriers, and its maximum concentrations form in the brain, a factor that provides for a high efficiency of the medicinal agent.

Likewise, to attain said result and provide the extended release of main components, according to a second alternative embodiment of the invention, a medicinal agent having a nootropic activity, comprising an active substance-dimethoshpone, further contains citric acid, lithium carbonate and a sugar syrup, with the following ratio of components, wt%:

| | |
|---|---|
| dimephosphone | 3.0-5.0 |
| lithium carbonate | 1.0-1.2 |
| citric acid | 4.0-5.0 |
| 64% sugar syrup | 89.0-92.0 |

The formulation sought for protection contains a reduced amount of dimephosphone, the increased quantity of a sugar syrup and can be recommended for treating children.

A sugar syrup has a complex of useful properties:
1) demonstrates a preserving effect. A high sugar concentration in syrups provides an osmotic pressure of a sugar solution greater than that of the cell fluid of micro-organisms. As a result of "drying" action of the solution, the conditions so obtained for vital activity of the micro-organisms are very adverse thereby to preclude souring and decay processes.
2) provides a sustained release of medicinal agents owing to a gradual release of dimephosphone and lithium ions from the sugar syrup.

According to a third alternative embodiment of the invention, a medicinal agent is in the form of a syrup and has an increased dimephosphone concentration. A homogeneous stable composition with an increased dimephosphone concentration is obtained by adding the sugar syrup with water. The medicinal agent further comprises deionized water, with the following ratio of components, wt%:

| | |
|---|---|
| dimephosphone | 14.0-30.0 |
| lithium carbonate | 1.0-3.0 |
| citric acid | 4.0-10.0 |
| 64% sugar syrup | 55.0-75.0 |
| water, deionized | up to 100 |

A stabilizing and preserving effect is provided by a greater dimephosphone concentration (14-30%) with a lesser sugar syrup concentration as against the second alternative embodiment.

Sugar syrups of this type can be recommended in the combination therapy of cerebral circulation disfunction as means weakening alcohol dependency and diminishing abstinence syndrome and improving metabolic processes in brain tissue.

Likewise, for said result to be achieved, according to a fourth alternative embodiment of the invention, a medicinal agent in the form of a syrup further comprises glycine and deionized water, with the following ratio of components, wt%:

| | |
|---|---|
| dimephosphone | 13.0-30.0 |
| lithium carbonate | 1.0-3.0 |
| citric acid | 4.0-11.0 |
| 64% sugar syrup | 45.0-75.0 |
| glycine | 2.0-5.0 |
| water, deionized | up to 100 |

Synergism of glycine, as a central retardation-type neuromediator, with dimephosphone and lithium ions, in the presence of citric acid is necessitated by the different mechanism of action of effective substances (lack of pharmacological incompatibility) and is affirmed by preclinical tests on rats. Besides, the glycine (aminoacetic acid) produces a stabilizing effect on a composition by the fixation of free lithium ions.

Active substances- dimephosphone, lithium carbonate, citric acid, glycine and a sugar syrup are available in medicinal agents in effective amounts. An amount less than the one as indicated produces no effect; an amount greater than the one as indicated is not to the purpose because no increment of the effect occurs.

### Best Modes of Carrying out the Invention

Table I cites examples illustrating the formulation of the claimed variants of medicinal agents having a nootropic and antidepressive activity. An amount of ingredients is given for 100 wt% of a composition.

Example I. Formulation of a medicinal agent, wt%:
dimephoshone - 15.0; citric acid - 3.0; lithium carbonate - 0.5; water, deionized - balance up to 100.

Method of preparation of agent. In a reactor provided with a water jacket and a stirrer, the calculated amounts of dimephosphone, deionized water and lithium carbonate are added one after another under heating and stirring, followed by the addition of the calculated quantitiy of citric acid to a suspension in small portions to control a step of foaming. The resultant clear mixture is of neutral reaction.

Examples 2-5 are carried out in line with a method, as shown and described in Example I.

For the formulation of compositions see Table 1.

Example 6. Formulation of a medicinal agent, wt%:
dimephoshone - 3.0; citric acid - 4.0; lithium carbonate - 1.0; 64% sugar syrup- 92.0.

Method of obtaining agents. In a copper tinplated, syrup -digesting reactor, provided with a stirrer, a 64% sugar syrup is prepared under Pharmacopoeia XI, as calculated in terms of 0.36 L of water for 0.64 kg of sugar, at a temperature comprised between 60 and 70°C for 40 minutes.

Density of a sugar syrup is 1.308-1.315 g/cm³. The calculated quantity of the sugar syrup is added with dimephosphone as required, heating to 90°C (the temperature of a water bath or a reactor jacket), adding lithium carbonate. A citric acid is then introduced in small portions under stirring and heating to control a step of foaming. The resultant syrup is a colorless clear, thick, odorless liquid; sweet-sour mountain ash taste, of neutral reaction.

Examples 7-8 are carried out in accordance with the method of Example 6. For formulations of medicinal agents see Table 1.

Examples 9-10 are carried out in accordance with the method of Example 6 by adding deionized water in the last step. For formulations of medicinal agents see Table I.

Examples 11-12 are carried out in accordance with the method of Example 6 by adding further deionized water and glycine in the last step, maintaining under heating and stirring to accomplish a complete dissolution of all the components. For formulation of medicinal agents see Table I.

The samples of medicinal agents - aqueous solution (Example 4) and syrups (Examples 9, 11) have been submitted to preclinical investigations. As experimental investigation has been conducted for the purpose of defining a specific effect produced by the medicinal agents on the indices of a behavioral activity of rats. The investigations have been carried out in accordance with a "Manual for Experimental (preclinical) Investigation of New Pharmacological Substances", - M.: MEDITSINA Publishers, 2005. 832 pages.

A pharmacological effect has been examined at the time of assessing the cognitive-mnestic peculiarities, behavioral activity and emotional conditions of rats with an intra-abdominal administration of medicinal agents: Examples 4, 9, 11 compared with the known preparation "Pyracetam". In an experiment, were used 50 mature white rats, Wistar's line, which constituted 5 experimental groups:
1. Medicinal agent (Example 4) having a volume of 2.8 ml;
2. Medicinal agent (Example 11) having a volume of 2.8 ml;
3. Medicinal agent (Example 9) having a volume of 2.8 ml;
4. Pyracetam having a volume of 1.5 ml;
5. Control - distilled water having a volume of 1.5 ml.

Evaluation of cognitive-mnestic peculiarities was made in a plant for producing a conditional reflex of passive adoidance (CRPA) "shuttle compartment" made up of two sections: a first dark section with an electrified grid floor and a second lit section, both separated by a movable closure. An experimental situation consisted of three steps.
1. Familiarization. A closure covering a lit section was opened, placing a rat in a lit compartment back to back a dark section (starting position) whereupon the closure was closed again. An animal turned before long and found an entry to the dark section to move there. A latent entering period was measured with a timer. The rat was removed, in 10 seconds, from the dark section to a residence cage. A procedure was repeated 3 times at 30-minutes intervals. A latent period of passing into the dark was measured the moment the animal had been placed in the compartment.
2. Training. The moment a rat entered a dark section, making the last attempt of familiarization, an electric shock was supplied -- 50 hertz, 1.5 mA for I s), placing the rat in a residence cage immediately. A medicinal agent was administered intra-abdominally by means of a probe 30 minutes before the start of training.
3. Reproduction. In 24 hours an animal was again placed in a lit half, establishing a period of entering the darkness. A test ended when the animal had entered a dark section or if the animal did not do it during 3 minutes.

A test of general motorial activity, orientation-investigating activity and emotional conditions of animals was performed in a plant "open field".

An animal was placed in a central square. On visual observation of the animal, separate behavioral acts were counted during a 5-minutes test. The behavioral indices of rats are as follows:
1. Vertical motorial activity (VMA) reflecting the orientation-investigating behavior of animals;
2. Horizontal motorial activity (HMA) reflecting a general motorial activity of rats.

For rats' emotional conditions to be assessed, the indices used are as follows:
3. dying away reaction period, s;
4. Duration of a grooming reaction, s;
5. Duration of a smelling reaction, s.

A medicinal agent was administered intra-abdominally using a probe, 30 minutes before the start of testing.

The results so obtained were processed on IBM PC/AT using application packages Statistics 6.0. A probability of differences of the indices, mean in the groups was determined using Student's t- test. The differences were considered to be significant at p < 0.05.

The evaluation results of cognitive-mnestic peculiarities of animals are shown in Table 2.

On check of CRPA retention in rats 24 hours after training, there was observed 100% CRPA reproduction in the group of animals, with Pyracetam administered.

In the groups with administration of medicinal agents Nos 4, 9, 11 there was observed CRPA reproduction; a latent transition period to a dark section on reproduction did not differ from a latent transition period to the dark section, of animals, with Pyracetam administered.

Experimental data obtainable at the time of testing in a plant "open field" allowing for assessing a behavioral activity of animals are cited in Table 3.

Under the conditions of an "open field, duration of a grooming reaction indicative of the restless condition of an animal in an "open field" situation unconventional to it is expressed in the control animals group. A reduced reaction period of grooming gives evidence of a well pronounced anxiolithic effect of the preparation and is utmost manifest in the groups: "Pyracetam", "Medicinal agent (Example 4), 2.8 ml in volume".

Experimental data on CRPA modeling on rats go to show that the action of medicinal agents as proposed is effective and comparable with the pharmacological effect of a standard preparation "Pyracetam".

For a comprehensive research study of effects exerted by medicinals (Examples 4, 9, 11) of the given concentration and form on cognitive and mnestic functions, use was made of a step of modeling a conditioned reflex of active avoidance (CRAA) to assess a degree of training of each and every individual animal, a phase of producing a conditioned reflex, in which the preparation starts exerting an optimizing effect, the emotional condition of the animal.

Evaluation of cognitive-mnestic peculiarities was made in a CRAA producing plant "shuttle compartment". The animals were previously tested to detect their ability to training. Rats with a low ability for training were detected by testing in a shuttle compartment made up of two similar sections with an electrode floor separated by a partition with a hole (Kozlovsky, Danchev, 2002). An unconditioned electric cutaneous aversive stimulus was applied to animal's paws after the previous isolated action of a code signal used as a sound of 4 s duration. Exciting current parameters: 50 hertz, 0.4 - 0.8 mA (in relation to individual sensitivity defined by the expression of a response). Intervals between representation of the code signal and the electric cutaneous stimulus - 2 s; maximum duration of each and every electric cutaneous stimulus - 4 s; interval between representations of code signals - 20 s. Conditioned reflex of active avoidance (CRAA) - transition to the adjacent section of the compartment during 6 s on connection of the code signal.

CRAA was produced during 5 days, 30 sets of stimuli daily. The rats which by the end of day of test training had over 15 insufficient or incomplete reactions were classified as inadequately trained (the animals with a low level of training). The rest of animals were divided into 5 groups, each including 8 animals.
1. Medicinal agent (Example 11), 2.8 ml;
2. Medicinal agent (Example 4), 2.8 ml;
3. Medicinal agent (Example 9), 2.8 ml;
4. Pyracetam, 1.5 ml;
5. Central - distilled water, 1.5 ml.

Training of animals was resumed 15 days after the course administration of preparations during 7 days and lasted 4 days. Preservation of a habit was determined 5 days after a training session.

At the time of making evaluation of the examination results of CRAA production dynamics, main behavioral indices were registered to characterize a process of animals training:
^{x} number of avoidance reactions (AR);
^{x} number of short-latent reactions of removing an electric cutaneous stimulus (less than I s) (SLR);
^{x} number of "incomplete" reactions (non-avoidance);
^{x} number of intersignal reactions (ISR);
^{x} latent time of avoidance (LTA).

The results thus obtained were processed on IBM PC/AT by means of application packages Statistica 6.0. Differences were considered significant at p < 0.05.

An influence exerted by test medicinal agents (Examples 4, 9, 11) on the dynamics of CRAA formation in rats - the number of avoidances (AR), the number of short-latent reactions of removing an electric cutaneous stimulus (less then I s) (SLR), the number of "incomplete" reactions (non-avoidance), the number of intersignal reactions (ISR), latent time of avoidance (LTA) are shown in Table 4. The footnotes of quantitative indices stand for:
* significant difference from Pyracetam, p < 0.05;
# significant difference from the initial level, p < 0.05;
S significant difference from the control, p < 0.05;
***-significant difference from the first day of training, p < 0.05.

The check results of preservation in rats, of a conditioned reflex produced, 5 days after the last training session (cf. Table 5) go to show a high level of reproduction of a conditioned reflex in the groups of mature animals with the administration of Pyracetam and test medicinal agents (Examples 4, 9, 11).

Likewise, submitted to preclinical investigations was a sample of medicinal No 7 (sugar syrups - child's form).

The pharmacological effects of the present medicinal agent were examined at the time of making evaluation of the orientation - investigating motorial activity, emotional conditions and memory trace restoration in a CRPA test.

Experiments were carried out on rats, aged 24-52 days, with a single intra-abdominal administration of 0.3-0.5 ml medicinal agent No 7 compared to the conventional preparation "Pyracetam". The number of animals in the control and test groups was 20 rats each, Wistar's line.

The investigating behavior of animals was tested in an "open field".

The "open field" we have used for little rats was a square platform, 900 cm in size, fenced by nontransparent walls, 14.5 cm high. A floor was traced in squares, 36 cm in size. The "open field" was evenly lit (150 lux) by an electrical lamp. The behavior of the little rats was tested during 3 minutes. The testing started with placing an animal in the center of the "open field". Registered were horizontal activity in the center (HAC) (the number of intersected squares) and horizontal activity in the periphery (HAP), vertical activity (VA) - the number of standing postures; the number of grooming acts (G).

Evaluation of cognitive-mnestic peculiarities was made in a plant for producing a conditioned reflex of passive avoidance (CRPA) "shuttle compartment" being comprised of two sections: a dark section with an electrified grid floor and a lit section both separated by a movable closure. Parameters of exciting current: 50 hertz, 0.4- 0.6 mA (depending on individual sensitivity being determined by the expression of a response). Duration of irritation - 4 s. Trigger stimulus is a stay in a bright illuminated space; main behavioural acts - passage to a dark room; unconditioned stimulus - current shock. Test was carried out in 3 steps:
1. Familiarization. An animal was seated back to back a dark room. For 15 s the animal was allowed to search a compartment and placed afterwards in a residential cage. A procedure was repeated thrice at a 30-minute interval.
2. Training. Upon entry in the dark room, 10 s after the last attempt to become familiar, a current shock was supplied after which the animal was shifted to a residential cage. Medicinal agents were administered intra-abdominally using a probe 30 minutes before the start of training.

3.Reproduction. It was performed on the next day after the training. An animal was again admitted to the compartment, and the time of entry in the dark room was measured by a timer.

The data so obtained were assayed by means of software packages STADIA. For comparison's sake use was made of Student's-test. Differences were considered significant at p < 0.05.

HAC and HAP indices in an "open field" in 24-day age rats in the groups with the administration of Pyracetam (11± 4.00; 36.15 ± 17.97) and medicinal No 7 (6.15±2.62; 26.35±7.86) adequately differed from the indices of the control group (2.80±2.46; 11.90±6.22). The significance of differences in the experimental and control groups, of the indices of vertical activity and grooming were evaluated. VA in the experimental groups was respectively 9.85±4.15 and 7.10±2.67 and 1.65±4.46 in the control groups. The sum total of acts of grooming in the experimental groups was 2.10±0.97 and 2.75±1.48, respectively, and 0.95±0.89, in the control.

The test animals demonstrated in an "open field" higher indices of horizontal activity (in the center and in the periphery), vertical activity (number of standing postures), a factor indicative of a good level of their orientation investigating activity. The grooming indices gave evidence of a reduced level of anxiety, disturbance of the animals - this also was a positive trait of the emotional status of the test animals.

A comparison of the results obtained in testing the mnestic functions in rats, aged 48-52 days, goes to show that at the time of training there was a significant difference of the latent period (LP) of transition to the dark section of a compartment in comparison with the control in animal groups with the administration of Pyracetam and medicinal No 7. On reproduction of CRPA in the test animal groups, the latent period of entry in the dark section appreciably increased, as compared to the same indices in training. Thus, while reproducing a CRPA habit in the animals which received medicinal No 7, the latent period (LP) significantly tended to increase from 12.40±2.42 s to 36.94±18.72 s, as compared to the same indices in the control animals - 8.68±2.67 s and 9.52±4.15 s. Pyracetam produced anti-amnestic effects significantly increasing relative to the control of LP transition to the dark compartment from 14.20±3.00 s to 44±19.27 s on reproduction of the CRPA in the animals. In the experimental animal group, with the administration of medicinal No 7, LP transition to the dark section, on reproduction, did not differ from the LP of transition to the dark section of the animals when Pyracetam is administered.

Experimental data obtained in an "open field" and on modeling CRPA on rats, aged 24-52 days, go to show that the action of medicinal agent No 7, as offered, (child's form) is effective and comparable with the pharmacological effect of a standard preparation "Pyracetam".

It is hence logical to see that the proposed group of effective medicinals in the form of aqueous solutions and syrups can perform particularly well in normalizing the physiological and functional activity of the central nervous system, intellectually-mnestic functions of the brain. The medicinal agents have more physiologically effective pH values, as shown in a wide range of concentrations, feature stability of solutions to dilution, have pleasant taste, which fact simplifies their use in child's practice.

**Table 1**

| Example | Formulation, wt% | | | | | |
|---|---|---|---|---|---|---|
| | Dimephosphone | Citric acid | Lithium carbonate | Sugar syrup | Glycine | Water |
| Mixture, aqueous. Aqueous solutions | | | | | | |
| 1 | 15,0 | 3,0 | 0,5 | | | up to 100 |
| 2 | 20,0 | 2,7 | 0,5 | | | Up to 100 |
| 3 | 28,3 | 2,4 | 3,5 | | | Up to 100 |
| 4 | 28,6 | 3,8 | 0,7 | | | Up to 100 |
| 5 | 30,0 | 4,0 | 5,0 | | | Up to 100 |
| Sugar syrups, 64% (child's form) | | | | | | |
| 6 | 3,0 | 4,0 | 1,0 | 92,0 | | |
| 7 | 3,7 | 4,4 | 1,1 | 90,8 | | |
| 8 | 5,0 | 5,0 | 1,0 | 89,0 | | |
| Sugar syrups with lesser sugar concentration | | | | | | |
| 9 | 14,3 | 4,3 | 1,1 | 75,0 | | up to 100 |
| 10 | 27,0 | 8,2 | 3,0 | 56,5 | | up to 100 |
| Sugar syrups with glycine | | | | | | |
| 11 | 13,7 | 4,1 | 1,0 | 71,0 | 2,0 | up to 100 |
| 12 | 30,0 | 10,3 | 3,0 | 48,7 | 5,0 | up to 100 |

**Table 2**

| Groups | Experiment steps | |
|---|---|---|
| | Training | Reproduction |
| | Latent period of passage to dark section s, M±σ | Latent period of passage to dark section s, M±σ |
| Medicinal No 11, 2,8 ml | 32,86±15,77 | 116,63±30,93* |
| Medicinal No 4, 2,8 ml | 29,00±6,18 | 140,88±25,83* |
| Medicinal No 9, 2,8 ml | 28,38±14,00 | 130,86±24,94* |
| Pyracetam | 44,75±9,41* | 180,0±0,00* |
| Control | 15,75±8,35 | 116,63±27,67*^{#} |

| | | |
|---|---|---|
| ^{x} Significan difference of the latent period of transition on reproduction from the latent period of transition to a dark section in training, p < 0.05; ^{xx} Significan difference of the latent period of transition to a dark section in training from the control in training, p < 0.05 ^{#} Significan difference of the latent period of transition to a dark section on reproduction from the latent period of transition to the dark section of animals when Pyracetam is administered, p < 0.05. | | |

**Table 3**

| | VMA M±m | HMA M±m | Duration of dying-away reaction, s M±m | Duration of grooming, s M±m | Duration of smelling reaction s M±m |
|---|---|---|---|---|---|
| Medicinal No 11, 2,8 ml | 4,50±1,05 | 80,50±16,74 | 144,38±32,49* | 21,25±12,46* | 21,13±5,67* |
| Medicinal No 4, 2, 8 ml | 1,50±0,80* | 38,13±16,88* *** | 189,50±30,12* | 6,63±3,41* | 26,50±11,39 |
| Medicinal No 9, 2,8 ml | 2,75±1,79 | 73,13±20,03 | 187,50±23,60* | 18,63±7,97* | 27,13±6,88 |
| Pyracetam | 3,13±2,02* | 34,37±17,65 | 161,25±45,50* | 13,63±6,94* | 27,88±8,68 |
| Control | 9,19±1,69 | 77,56±9,14 | 52,0±18,06 | 44,81±6,89 | 46,94±9,80 |

| | | | | | |
|---|---|---|---|---|---|
| * - significant difference from the control, p < 0,05; ** - significant difference from Pyracetam, p < 0,05; *** - significant difference of medicinal No 4 from medicinal No 11, p < 0,05; | | | | | |

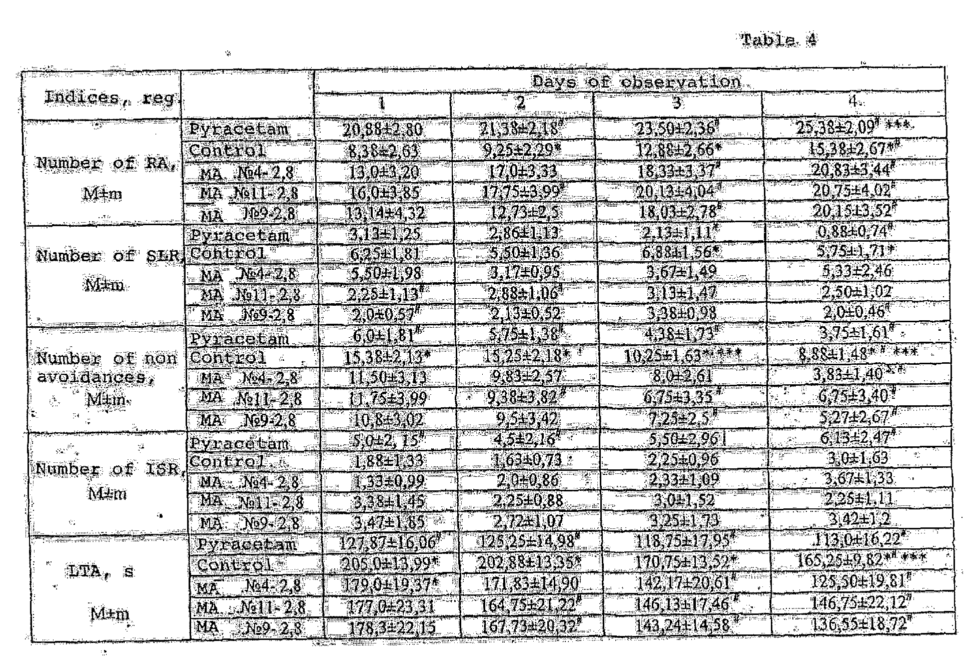

**Table 5**

| Substance | Reg. indices | |
|---|---|---|
| | Number of RA, M±m | LTA, s M±m |
| Pyracetam | 26,00±1,39 | 111,13±11,52 |
| Control | 17,88±7,75* | 156,38±15,73* |
| Medicinal agent No 4, 2,8 | 21,00±3,75 | 124,50±26,85 |
| Medicinal agent No 11, 2,8 | 22,63±3,77 | 127,50±22,89 |
| Medicinal agent No 9, 2,8 | 23,72±3,87 | 126,50±24,69 |

| | | |
|---|---|---|
| *- significant difference from Pyracetam, p < 0.05; | | |

## Claims

1. A medicinal agent having a nootropic activity, comprising an active substance "Dimephosphone" and water, **characterized in** further comprising citric acid and lithium carbonate, with the following ratio of components, wt%;
Dimephosphone 15.0 - 30.0
Lithium carbonate 0.5-5.0
Citric acid 3.0-4.0
Water, deionized up to 100

2. A medicinal agent having a nootropic activity, comprising an active substance "Dimephosphone", **characterized in** further comprising citric acid, lithium carbonate and sugar syrup, with the following ratio of components, wt%;
Dimephosphone 3.0 - 5.0
Lithium carbonate 1.0-1.2
Citric acid 4.0-5.0
64% sugar syrup 89.0-92.0
Water, deionized up to 100

3. A medicinal agent having a nootropic activity, comprising an active substance "Dimephosphone" and water, **characterized in** further comprising citric acid, lithium carbonate and sugar syrup, with the following ratio of components, wt%;
Dimephosphone 14.0 - 30.0
Lithium carbonate 1.0-3.0
Citric acid 4.0-10.0
64% sugar syrup 55.0-75.0
Water, deionized up to 100

4. A medicinal agent having a nootropic activity, comprising an active substance "Dimephosphone" and water, **characterized in** further comprising citric acid, lithium carbonate, sugar syrup and glycine, with the following ratio of components, wt%;
Dimephosphone 13.0 - 30.0
Lithium carbonate 1.0-3.0
Citric acid 4.0-11.0
64% sugar syrup 45.0-75.0
Glycine 2.0-5.0
Water, deionized up to 100
